(19) Europäisches Patentamt

European Patent Office

Office européen des brevets

(11) **EP 1 201 194 A2**

(12) **EUROPÄISCHE PATENTANMELDUNG**

(43) Veröffentlichungstag:
02.05.2002 Patentblatt 2002/18

(51) Int Cl.⁷: **A61B 17/32**, A61B 17/58

(21) Anmeldenummer: 01119579.9

(22) Anmeldetag: 16.08.2001

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **27.10.2000 DE 20018390 U**

(71) Anmelder: **Wenzler Medizintechnik GmbH**
**78665 Frittlingen (DE)**

(72) Erfinder: **Wenzler, Peter**
**78665 Frittlingen (DE)**

(74) Vertreter: **Neymeyer, Franz, Dipl.-Ing. (FH)**
**Haselweg 20**
**78052 Villingen-Schwenningen (DE)**

(54) **Chirurgische Schneidzange**

(57) Die Schneidzange dient zum Durchtrennen von Drähten (40) oder dgl. Sie weist einen ersten zweiarmigen Griffhebel (1) auf, dessen kürzerer vorderer Hebelarm (3) an seinem vorderen Endabschnitt mit einer Schneide versehen ist. Ein kurzer Hebelarm (11) eines zweiten Griffhebels (10) ist über ein Hebelgetriebe mit einem die zweite Schneide aufweisenden zweiarmigen Schneidhebel (20) getrieblich verbunden. Um weniger Gelenke zu benötigen und höhere Progressionen der Schneidkräfte am Schneidhebel beim Schneidvorgang zu erzielen, ist der kurze Hebelarm (11) des zweiten Griffhebels (10) in einem radialen Abstand vom Zangengelenk (Z) mit einer Kurvenbahn ($K_1,K_2$) versehen, über welche dieser kurze Hebelarm (11) auf den Schneidhebel (20) einwirkt. Zu diesem Zweck ist dieser Hebelarm (25) mit einem auf der Kurvenbahn ($K_1,K_2$) gleitenden Übertragungselements (31,31') versehen. Die Kurvenbahn ($K,K_1,K_2$) verläuft so, daß sich der radiale Abstand ($a_0,a_1,a_2$), den die Kraftlinien ($Kl_0,KL_1,KL_2$) der während einer Schließbewegung des zweiten Griffhebels (10) jeweils im Kraftübertragungspunkt ($E_1,E$) an der Kurvenbahn ($K,K_1,K_2$) wirksamen Schneidkraftkomponente von der Achse (S) des Zangengelenks (Z) aufweisen, von einem Maximalwert ($a_0$) am Beginn der Schließbewegung in einen Minimalwert ($a_2$) am Ende der Schließbewegung ändert.

Fig. 1

EP 1 201 194 A2

**Beschreibung**

Die Erfindung betrifft eine Schneidzange, insbesondere für chirurgische Zwecke, zum Durchtrennen von Drähten, Nägeln, Schrauben oder dgl. mit einem ersten zweiarmigen Griffhebel, dessen längerer hinterer Hebelarm als Handgriff ausgebildet ist und dessen kürzerer vorderer Hebelarm an seinem vorderen Endabschnitt mit einer Schneide versehen ist, und mit einem ebenfalls zweiarmigen zweiten Griffhebel, der durch ein Zangengelenk schwenkbar am ersten Griffhebel angelenkt ist und der außer einem zum Handgriff des ersten Griffhebels etwa spiegelbildlich ausgebildeten und angeordneten zweiten Handgriff einen vorderen kurzen Hebelarm aufweist, der über ein Hebelgetriebe mit einem eine zweite Schneide od. dgl. aufweisenden zweiarmigen, am kurzen Hebelarm des ersten Griffhebels angelenkten Schneidhebel derart getrieblich verbunden ist, daß die über die Handgriffe auf die Schneiden ausgeübte Schneidkraft mit zunehmender Schließbewegung überproportional ansteigt.

Aus EP 0 321 884 B1 ist bereits eine Zange der gattungsgemäßen Art für chirurgische Zwecke zum Durchtrennen von Drähten, Schrauben od. dgl. bekannt.

Diese Zange ist mit einem Schneiden-Griffhebel versehen, der an seinem vorderen Ende eine Schneide und an seinem hinteren Ende einen Handgriff aufweist. An diesem Schneiden-Griffhebel ist mittels eines Schneidenhebelgelenks ein Schneidenhebel gelagert, der eine zweite, der ersten Schneide gegenüberliegende Schneide aufweist. Außerdem ist an dem Schneiden-Griffhebel mittels eines Griffhebelgelenks schwenkbar ein Griffhebel angelenkt, wobei zwischen dem Griffhebel und dem Schneidenhebel ein Getriebe angeordnet ist, von dem ein Antriebsglied vom Griffhebel beaufschlagt wird. Dabei greift das Antriebsglied des Getriebes auf der von der Schneide abgewandten Seite eines den Schneidenhebel am Schneiden-Griffhebel abstützenden Schneidenhebelgelenks am Schneidenhebel an, wobei gleiche Relativverschwenkungen des Griffhebels gegenüber dem Schneiden-Griffhebel eine deutlich geringer werdende Verschwenkung des Schneidenhebels gegenüber dem Schneiden-Griffhebel bewirken, so daß die Kraftübersetzung automatisch mit kleiner werdendem Abstand zwischen den jeweils am Griffhebel und am Schneiden-Griffhebel ausgebildeten Handgriffen zunimmt. Das bedeutet, daß sich die Hebelübersetzung mit zunehmender Schließbewegung in der Weise ändert, daß einer gegebenen Winkelbewegung des einen Griffhebels zum andern hin, eine immer geringer werdende Winkelbewegung der bewegten Schneide zur anderen Schneide hin stattfindet. Man erreicht durch diese Übersetzungsänderung, die sich zwangsläufig während der Schließbewegung ergibt, bei gleichbleibender Schließkraft am Griffhebel eine zunehmende Schneidkraft an der bewegten Schneide.

Zurückzuführen ist diese Übersetzungsänderung auf die Veränderung des die Schneidbewegung der Schneide bewirkenden Hebelarms.

Die Veränderung des wirksamen Hebelarms wird bei einer Ausführungsform der bekannten Zange durch eine Art Kniegelenk erreicht. Dabei ist ein kurzer Hebelarm des schwenkbeweglich am zweiten Hebelarm gelagerten ersten Hebelarms durch eine Lasche gelenkig mit einem längeren Hebelarm eines zweiarmigen Schneidenhebels, der ebenfalls am zweiten Hebelarm des Griffhebels schwenkbar gelagert ist, verbunden. Es sind somit bei dieser bekannten Zange insgesamt vier Gelenke vorgesehen. Bei den zeichnerisch dargestellten Hebelverhältnissen ergibt sich eine Veränderung der Schneidkraft während der gesamten Schließbewegung, die einem Faktor von etwa 1,7 entspricht. Das bedeutet, daß die anfängliche Schneidkraft bis zum Ende der Schließbewegung um den Faktor 1,7 zunimmt.

Bei einer anderen Ausführungsform dieser bekannten Zange ist am ersten Griffhebel, dessen zweiter kurzer Arm mit der ersten Schneide versehen ist und an dem schwenkbar ein zweiarmiger Schneidenhebel sowie der zweite Griffhebel schwenkbar gelagert sind, ein Kniehebel schwenkbar gelagert. Dieser Kniehebel steht über ein Gelenk mit einer Schubstange in Verbindung, die ihrerseits mit dem anderen. Ende am beweglichen Griffhebel angelenkt ist. Der Kniehebel steht über ein Verbindungsglied mit dem längeren Hebelarm des Schneidhebels gelenkig in Verbindung. Bei dieser Anordnung ergeben sich während der Schließbewegung Änderungen bezüglich zweier wirksamer Hebelarme, nämlich des wirksamen Hebelarms an dem Kniehebel und des wirksamen Hebelarms am Schneidenhebel.

Bei dieser Ausführungsform benötigt man insgesamt sechs Gelenke und erreicht bei den dargestellten Hebelverhältnissen der Zeichnung eine Vergrößerung der Schneidkraft um etwa den Faktor 28.

Während die Ausführungsform mit den vier Gelenken im wesentlichen aus vier beweglich miteinander verbundenen Teilen besteht, weist die andere Ausführungsform dieser bekannten Zange mit den zwei Kniegelenken sechs gelenkig miteinander verbundene Teile auf.

Der Erfindung liegt die Aufgabe zugrunde, eine Schneidzange der eingangs genannten Art zu schaffen, die aus möglichst wenig Teilen besteht, insbesondere weniger Gelenke benötigt als die bekannten Schneidzangen, und mit welcher auch höhere bzw. unterschiedliche Progressionen der Schneidkräfte innerhalb eines Schneidvorgangs erzielbar sind.

Gelöst wird diese Aufgabe erfindungsgemäß dadurch, daß der kurze Hebelarm des zweiten Griffhebels in einem radialen Abstand vom Zangengelenk eine Kurvenbahn aufweist, über welche dieser kurze Hebelarm während eines Schneidvorgangs auf den ihm zugekehrten Hebelarm des Schneidhebels einwirkt, der zu diesem Zweck mit einem auf der Kurvenbahn reibungsarm rollenden oder gleitenden Übertragungselements versehen ist, und daß die Kurvenbahn so verläuft, daß sich der radiale Abstand, den die Kraftlinie der während einer Schließbewegung des zweiten Griffhebels jeweils im Berührungspunkt der Rolle mit der Kurvenbahn wirksamen Schließkraftkomponente von der Achse des Zangengelenks aufweist, von einem Maximalwert am Beginn der Schließbewegung in einen Minimalwert am Ende der Schließbewegung ändert.

Abgesehen davon, daß die erfindungsgemäße Schneidzange nur aus insgesamt drei gelenkig miteinander verbundenen Zangenteilen besteht und nur zwei Gelenke sowie eine Kurvenbahn aufweist, lassen sich mit dem der Erfindung zugrunde liegenden Konstruktions- bzw. Funktionsprinzip auf einfache Weise Schneidzangen herstellen, bei denen sich die Schneidkräfte, die das Abtrennen eines Drahtes, einer Schraube od. dgl. bewirken, den Wünschen der Anwender individuell anpassen lassen. Es ist dazu lediglich erforderlich, der Kurvenbahn einen entsprechenden Verlauf zu geben, wobei die Möglichkeit besteht, der Kurvenbahn verschiedene Neigungen zu geben, welche sich unterschiedlich auf die Progressivität der Schneidkrafterhöhung auswirken.

Vor allem ist es möglich, durch die entsprechende Gestaltung der Kurvenbahn die größte Progression der Kraftzunahme in jenen Bereich des Schließwinkels zu legen, in dem auch der Schneidwiderstand am größten ist.

Durch die Ausgestaltung nach Anspruch 2 wird eine einfache Standardausführung ermöglicht, bei der die Progression innerhalb des Schließwinkels bzw. über den gesamten Verlauf der Kurvenbahn an einer vom Schließwinkel abhängigen Sinusfunktion verläuft, wobei die Möglichkeit besteht, den günstigsten Verlaufsbereich dieser Sinusfunktion, beispielsweise den mit der größten Steigung, auszuwählen.

Mit den Ausgestaltungen nach den Ansprüchen 3 bis 6 läßt sich die erfindungsgemäße Schneidzange auf sehr einfache und kostengünstige Weise herstellen. Außerdem ist eine solche Zange sehr einfach zu handhaben.

Statt der gemäß Anspruch 6 vorgesehenen Rolle als Übertragungselement, kann auch die Ausgestaltung nach Anspruch 7 vorgesehen sein, die einen Kulissenstein als Übertragungselement vorsieht.

Die Ausgestaltungen nach den Ansprüchen 8 bis 11 betreffen ein Ausführungsbeispiel, bei dem die Kurvenbahn keine stetige Steigung und keinen stetigen Verlauf und somit auch keine stetige Progressivität über ihre Gesamtlänge aufweist. Diese Kurvenbahn besteht vielmehr aus zwei Abschnitten mit unterschiedlichen Steigungen bzw. Krümmungsradien, die so gewählt sind, daß an dem etwa in der Mitte der Kurvenbahn liegenden Ende des ersten Abschnitts die größte Kraftzunahme an den Schneiden erreicht wird, während die Progression im Verlauf des weiteren Schließwinkels bzw. des zweiten Abschnitts schwächer wird.

Anhand der Zeichnungen wird die Erfindung im folgenden näher erläutert. Es zeigt:

Fig. 1 die Seitenansicht einer Schneidzange im geöffneten Zustand;

Fig. 2 eine teilweise geschnittene Ansicht II aus Fig. 1;

Fig. 3 einen Schnitt III-III aus Fig. 1;

Fig. 4 die schmalseitige Ansicht des angelenkten zweiten Griffhebels;

Fig. 5 den Griffhebel der Fig. 4 in Seitenansicht;

Fig. 5a in vergrößerter Darstellung den Kopfteil des Griffhebels der Fig. 5;

Fig. 6 eine schmalseitige Ansicht VI des Schneidhebels aus Fig. 1;

Fig. 7 eine schmalseitige Ansicht VII des ersten Griffteils;

Fig. 8 eine Seitenansicht der Schneidzange in geschlossenem Zustand;

Fig. 8a in schematischer Darstellung die Hebelverhältnisse der Schneidzange am Beginn einer Schneidbewegung;

Fig. 8b in schematischer Darstellung die Hebelverhältnisse der Schneidzange am Ende einer Schneidbewegung;

Fig. 9 in schematisch vereinfachter aber vergrößerter Darstellung die Kulissenführung mit der am Anfang der Kurvenbahn auf das als Rolle ausgebildete Übertragungselement wirksamen Kraftlinie;

Fig. 10 in schematisch vereinfachter aber vergrößerter Darstellung die Kulissenführung mit der in der Mitte der Kurvenbahn auf das als Rolle ausgebildete Übertragungselement wirksamen Kraftlinie;

Fig. 11 in schematisch vereinfachter aber vergrößerter Darstellung die Kulissenführung mit der am Ende der Kurvenbahn auf das als Rolle ausgebildete Übertragungselement wirksamen Kraftlinie;

Fig. 12 in schematisch vereinfachter aber vergrößerter Darstellung die Kulissenführung mit dem als Rolle ausgebildete Übertragungselement;

Fig. 13    in vergrößerter Darstellung eine andere Form der Kulissenführung mit einem Gleitstein als Übertragungselement;

Fig. 14    den Gleitstein aus Fig. 13;

Fig. 15    in schematischer Darstellung die Winkel- und Kraftarme die am Zangengelenk bzw. an einer Kurvenbahn mit gleichförmiger Krümmung wirksam sind;

Fig. 16    eine schematische Darstellung der Kurvenbahn aus Fig. 15 im bezug auf das Zangengelenk;

Fig. 17    in schematischer Darstellung die am Zangengelenk wirksamen Kraftarme bei einer Kurvenbahn mit unterschiedlichen Krümmungen;

Fig. 18    die Kurvenbahn aus Fig. 17 im bezug auf das Zangengelenk.

Die in der Zeichnung dargestellte Schneidzange, die für chirurgische Zwecke entwickelt wurde und zum Durchtrennen von Drähten, Nägeln, Schrauben od. dgl. chirurgischer Hilfsmittel dient, weist einen ersten zweiarmigen Griffhebel 1 auf, dessen längerer hinterer Hebelarm 2 als Handgriff ausgebildet ist und dessen kürzerer, vorderer Hebelarm 3 an seinem vorderen Endabschnitt mit einem auswechselbaren Schneidplättchen 4 versehen ist.

Ein zweiter, ebenfalls zweiarmiger Griffhebel 10, ist mittels eines Zangengelenks Z schwenkbar am ersten Griffhebel 1 angelenkt. Dieser zweite Griffhebel 10 weist ebenfalls einen längeren Hebelarm 12 auf, der als Handgriff ausgebildet ist, wobei dieser Handgriff des zweiten Griffhebels 10 etwa spiegelbildlich zum Handgriff des ersten Griffhebels 1 ausgebildet und angeordnet ist.

Ein vorderer kurzer Hebelarm 11, der die Form einer dünneren Platte aufweist, die in einer entsprechend ausgebildeten schlitzartigen Ausnehmung 13 des ersten Hebelarms 1 schwenkbar geführt ist, besitzt eine Gelenkbohrung 14. Mit dieser Gelenkbohrung 14 ist der kurze Hebelarm 11 des zweiten Griffhebels 10 auf einem Gelenkbolzen 15 des ersten Griffhebels 1 schwenkbar gelagert. Der Gelenkbolzen 15 und die Gelenkbohrung 14 bilden das Zangengelenk Z.

Über diesen kurzen Hebelarm 11 steht der zweite Griffhebel 10 getrieblich in Verbindung mit einem zweiarmigen Schneidhebel 20, der mittels eines Gelenks G am kurzen Hebelarm des ersten Griffhebels 1 angelenkt ist. Zur schwenkbaren Aufnahme des plattenförmigen kurzen Hebelarms 11 des zweiten Griffhebels 12 ist auch der Schneidhebel 20 mit einer schlitzförmigen Ausnehmung 21 versehen. Außerdem ist auch das Lagerauge 22 des Gelenks G mit einer schlitzförmigen Ausnehmung 23 versehen, von welcher ein ebenfalls plattenförmiges Gelenkauge 24 des vorderen Hebels 3 des ersten Griffhebels 1 aufgenommen ist. Das Gelenk G ist gleich ausgebildet wie das Zangengelenk Z.

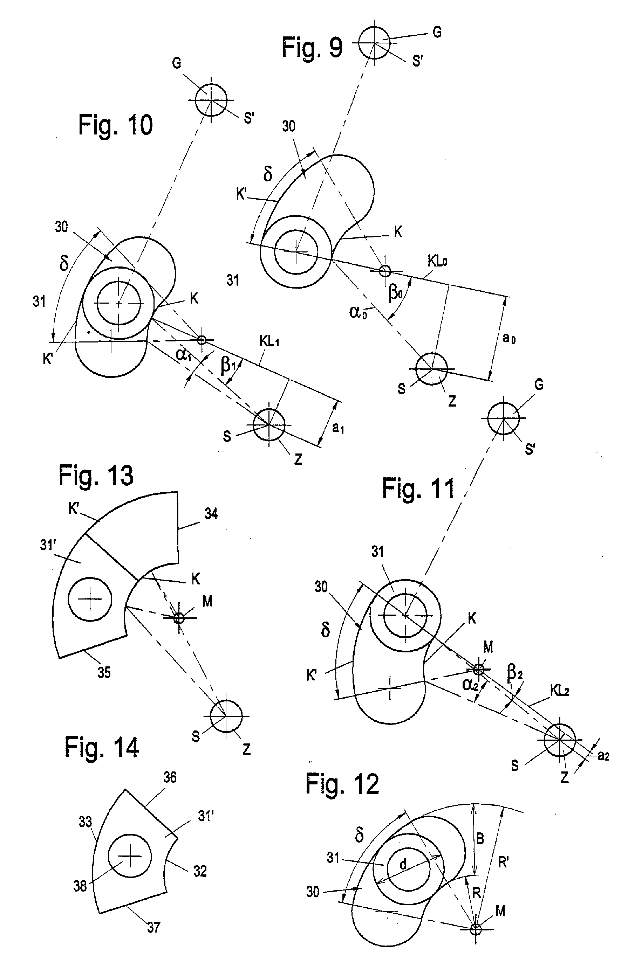

Die getriebliche Verbindung zwischen dem kurzen Hebelarm 11 des zweiten Griffhebels 10 und dem geschlitzten Hebelarm 25 des Schneidhebels 20 besteht aus einer Kulissenführung 30, die in Form eines gekrümmten Langlochs in den kurzen Hebelarm 11 eingearbeitet ist, sowie aus einer am Hebelarm 25 des Schneidhebels 20 auf einem eingeschraubten Lagerzapfen 33 gelagerten Rolle 31 als Übertragungselement. Dabei hat die Kulissenführung 30 eine Breite **B**, die dem Durchmesser **d** der Rolle 31 entspricht (Fig. 12).

Die Rolle 31 ist somit in der mit abgerundeten Enden versehenen Kulissenführung 30 in beiden Schwenkrichtungen des Hebelarms 11 zwangsgeführt.

Wie in den Fig. 13 und 14 dargestellt ist, kann statt einer Rolle 31 ein Kulissenstein 31' als Übertragungselement vorgesehen sein, dessen Gleitflächen 32 und 33 jeweils Krümmungen aufweisen, die den Krümmungen der Kurvenbahnen K und K' der in diesem Falle etwas längeren und mit radial verlaufenden Endflächen 34 und 35 versehenen Kulissenführung 30' angepaßt sind. Wie aus Fig. 14 ersichtlich ist, besitzt der Kulissenstein die Form eines Kreisringabschnitts mit radialen Endflächen 36 und 37 und einer Lagerbohrung 38.

Maßgebend für die Kraft- bzw. Bewegungsübertragung vom kurzen plattenförmigen Hebelarm 11 des zweiten Griffhebels 10 auf den Schneidhebel 20, dessen zweiter Hebelarm 26 mit den zweiten Schneidplättchen 27 versehen ist, ist die sich über einen Winkel δ erstreckende Kurvenbahn K. Diese Kurvenbahn K ist in einem gewissen Abstand vom Zangengelenk Z so angeordnet und mit einem solchen Verlauf versehen, daß sich der radiale Abstand a, den die Kraftlinien $KL_0$, $KL_1$ bzw. $KL_2$ während der Schließbewegung des zweiten Griffhebels 10 jeweils im Kraftübertragungspunkt an der Kurvenbahn K wirksamen Schneidkraftkomponente von der Achse S des Zangengelenks Z aufweisen, von einem Maximalwert ($a_0$) am Beginn der Schließbewegung in einen Minimalwert ($a_2$) am Ende der Schließbewegung ändert.

Diese sich ändernden Abstände $a_0$, $a_1$ und $a_2$ sind identisch mit dem jeweils wirksamen Hebelarm, an welchem die Kraft vom kurzen Hebelarm 11 des Griffhebels 10 auf den Schneidhebel 20 übertragen wird. In Folge der Krümmung der Kurvenbahn K ergibt sich während der Schließbewegung diese beschriebene Änderung des wirksamen Hebelarms $a_0$ nach $a_2$. Wichtig ist dabei auch, daß der Anfangspunkt A, der am Beginn der Schließbewegung mit der Rolle 31 in Berührung steht, von der Achse S des Zangengelenks Z einen kleineren Abstand $s_0$ aufweist als der Endpunkt E mit dem Abstand $s_2$.

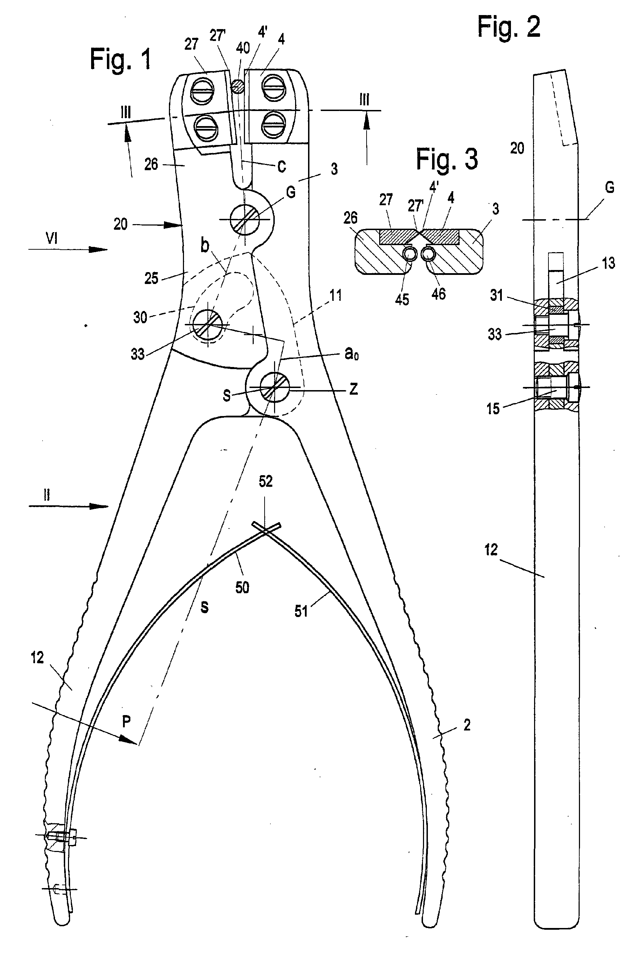

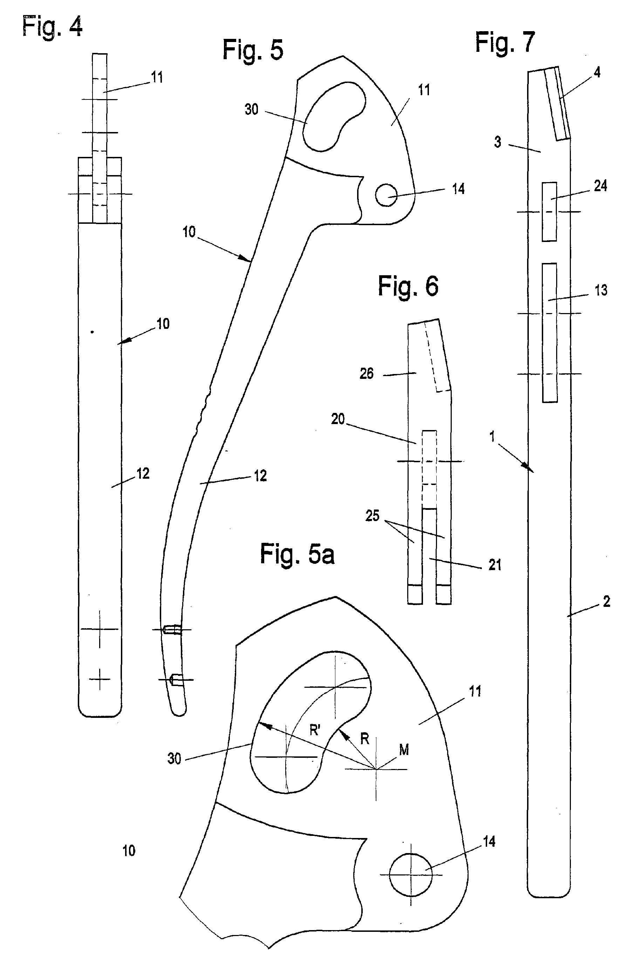

Im gezeichneten Ausführungsbeispiel gemäß den Fig. 1 bis 16 ist der Abstand $s_2$ etwa um 10% bis 15% größer als der Abstand $s_1$. Durch die konvexe Form der Kurvenbahn K sind alle zwischen A und E liegenden Abstände der Kurvenbahn K größer als $s_0$.

Bei dieser Ausführungsform weist die Kurvenbahn K eine konstante Krümmung auf, deren Krümmungsmittelpunkt M auf der vom Hebelarm 12 abgewandten Seite der das Ende E der Kurvenbahn K mit der Achse S des Zangengelenks Z verbindenden Geraden ES liegt. Der Krümmungsradius R der Kurvenbahn K ist wenigstens um ein Drittel kleiner als die Länge dieser Geraden ES bzw. der Abstand $s_2$.

Im bevorzugten Ausführungsbeispiel entspricht der Krümmungsradius R der Kurvenbahn K etwa der halben Länge der Geraden ES bzw. dem halben Abstand $s_2$, den der Endpunkt E der Kurvenbahn K von der Achse S des Zangengelenks Z hat.

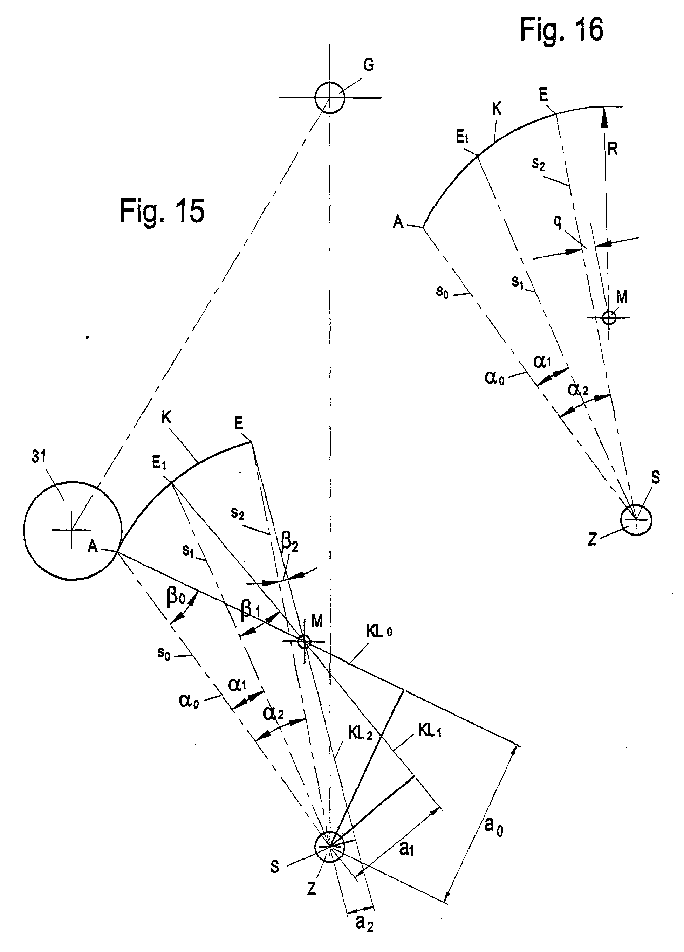

Wie aus Fig. 16 ersichtlich ist, hat der Krümmungsmittelpunkt M von der Geraden ES einen Abstand q, der prinzipiell beliebig gewählt werden könnte, der aber maßgebend ist für die Größe des wirksamen Hebelarms $a_0$ am Beginn der Schließ- bzw. Schneidbewegung und ebenso für den kleinstmöglichen Hebelarm $a_2$ am Ende der Schließbewegung.

Wenn man diesen Krümmungsmittelpunkt M direkt auf die Gerade ES legt, wird der am Ende der Schließbewegung wirksame Hebelarm $a_2$ gleich null. Theoretisch würde dadurch die Schneidkraft unendlich. Praktisch findet in einem solchen Fall aber keine Bewegungsübertragung mehr statt, was bedeutet, daß der Krümmungsmittelpunkt M immer einen Mindestabstand von der Geraden ES haben muß. Bei einer bevorzugten Ausführungsform entspricht dieser Abstand q etwa einem Zwölftel des Krümmungsradius R der Kurvenbahn K. In jedem Falle sollte dieser Abstand q kleiner sein als ein Zehntel des Krümmungsradius R, damit auch am Ende der Schließbewegung noch eine Bewegungsübertragung bzw. Kraftübertragung stattfinden könnte bzw. kann.

Wie sich die zwischen der Kurvenbahn K und der Rolle 31 am kurzen Hebelarm 11 des zweiten Griffhebels 10 wirksamen Hebelarme $a_0$, $a_1$ und $a_2$ während der Schließbewegung über den Winkel $\alpha_2$ ändern, ist in den Fig. 9, 10 und 11 bzw. 15 an drei Bewegungswinkeln $\alpha_0$, $\alpha_1$, $\alpha_2$ dargestellt. Diesen Bewegungswinkeln $\alpha_0$, $\alpha_1$, $\alpha_2$ des zweiten Griffhebels 10 und somit auch der Kurvenbahn K des kurzen Hebelarms 11 entsprechen die jeweiligen Winkel $\beta_0$, $\beta_1$, $\beta_2$, welche die jeweiligen Kraftlinien $Kl_0$ bzw. $KL_1$ und $KL_3$ in den Berührungspunkten A, $E_1$ bzw. E mit den Linien bzw. Geraden AS, $E_1S$, ES bilden, welche diese Berührungspunkte A, $E_1$ und E mit der Achse S des Zangengelenks Z verbinden.

Man erkennt in den Fig. 15 und 16 zudem, daß sich auch die Längen dieser Verbindungsgeraden AS bzw. $E_1S$ bzw. ES im Sinne einer stetigen Vergrößerung verändern, was schließlich zu der Auslenkbewegung bzw. Schneidbewegung des Schneidhebels 20 führt, die von der Kurvenbahn K über die Rolle 30 auf diesen ausgeübt wird.

Aus den Fig. 9 bis 12 ist erkennbar, daß die Kurvenbahn K mit einer zweiten dazu parallelen Kurvenbahn K' die Kulissenführung 30 bildet, die bei der Verwendung einer Rolle 31 als Übertragungselement in zweckmäßiger Weise mit abgerundeten Enden versehen ist.

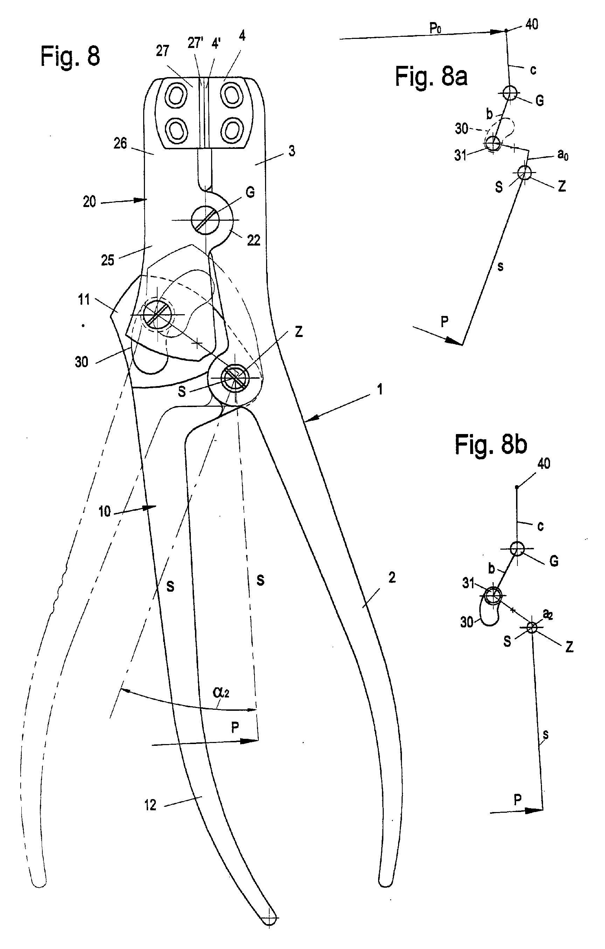

Bei den einer praktischen Ausführungsform der erfindungsgemäßen Schneidzange entsprechenden Hebelverhältnissen, die in den Fig. 8a und 8b schematisch dargestellt sind, errechnen sich die beim Abschneiden eines Drahtes 40 (Fig. 1) auftretenden Schneidkräfte $P_0$, $P_1$ und $P_2$, die bei den in Fig. 15 dargestellten Bewegungswinkeln $\alpha_0$, $\alpha_1$ und $\alpha_2$ auftreten wie folgt:

$$P_0 = \frac{s}{a_0} * \frac{c}{b}$$

$$P_1 = \frac{s}{a_1} * \frac{c}{b}$$

$$P_2 = \frac{s}{a_2} * \frac{c}{b}$$

Dabei ist s der Hebelarm, an dem die manuelle Kraft P des Benutzers wirksam ist; $a_0$, $a_1$, $a_2$ sind jeweils die Hebelarme die sich mit zunehmender Schließbewegung ändern und zwischen dem kurzen Hebelarm 11 und dem Schneidhebel 20 wirksam sind; b ist der Hebelarm zwischen dem Gelenk G und der Rolle 31, und c ist der Hebelarm zwischen dem Gelenk G und dem abzuschneidenden Draht 40.

Geht man von einer manuellen Kraft P = 100 N, s = 100 mm, $a_0$ = 10,5 mm, $a_1$ = 7 mm, $a_2$ = 1,66 mm, b = 30 mm und c = 35 mm aus, so ergeben sich aus den obigen Formeln für die am Draht 40 wirksamen Schneidkräfte folgende Werte:

$P_0$ = 816,3 N, $P_1$ = 1224,5 N und $P_2$ = 5.163,5 N.

Man erkennt daraus, daß die Schneidkraft am Ende der Schließbewegung 6,33 mal größer ist als am Anfang der Schließbewegung, d.h. $P_2$ = 6,33 x $P_0$. Außerdem erkennt man, daß die Progression in der ersten Hälfte der Schließbewegung, also im Winkelbereich $\alpha_1$ wesentlich geringer ist als in der restlichen Hälfte des Schließwinkels $\alpha_2$.

Aus den Darstellungen der Fig. 9 bis 11 und 15 ist auch erkennbar, daß die über die Kurvenbahn K an der Rolle 31 wirksamen Hebelarme $a_0$, $a_1$, $a_2$ sich jeweils berechnen lassen nach der Formel $a_0 = s_0$ x sin $\beta_0$, $a_1 = s_1$ x sin $\beta_1$ und $a_2 = s_2$ x sin $\beta_2$, wobei $s_0$, $s_1$ und $s_2$ jeweils die Abstände der Achse S des Zangengelenks Z von dem betreffenden Berührungspunkt A bzw. E1 und E der Rolle 31 mit der Kurvenbahn K darstellen.

Die jeweiligen Winkelwerte $\beta_0$, $\beta_1$ und $\beta_2$ lassen sich über die jeweilige Beziehung zwischen $\alpha_0$, $\alpha_1$ und $\alpha_2$ zu dem Winkel $\delta$ rechnerisch ermitteln.

$\delta$ ist der Winkel des Kreissegments, das die Kurvenbahn K einschließt und den Krümmungsmittelpunkt M als Zentrum besitzt.

Wie bereits eingangs erwähnt, ist es bei diesem Konstruktionsprinzip auch möglich, eine Kurvenbahn mit unterschiedlichen Steigungen oder Krümmungen vorzusehen um unterschiedliche Progressionen der an den Schneiden 4' und 27' zur Verfügung stehenden Schneidkraft $P_0$ und $P_2$ zu erreichen.

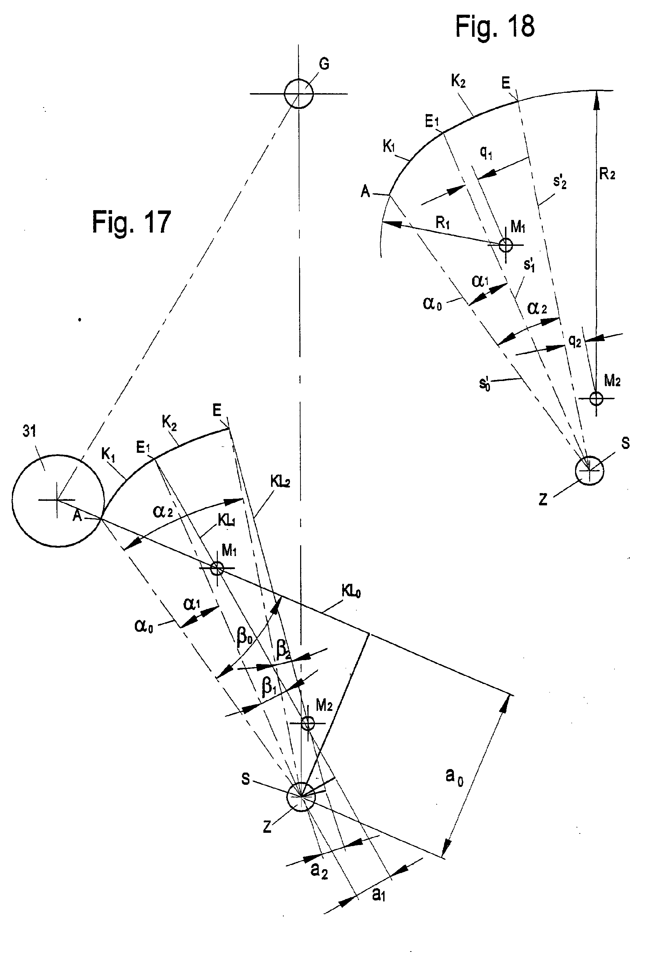

In den Fig. 17 und 18 ist ein solches Ausführungsbeispiel schematisch dargestellt. Die am Anfangspunkt A beginnende und am Endpunkt E endende Kurvenbahn besteht aus zwei Abschnitten $K_1$ und $K_2$, die unterschiedliche Krümmungsradien $R_1$ und $R_2$ und auch unterschiedliche Krümmungsmittelpunkte $M_1$ und $M_2$ aufweisen.

Der Krümmungsmittelpunkt $M_1$ der Kurvenbahn $K_1$ liegt auf der dem Hebelarm 12 des Griffhebels 10 gegenüberliegenden Seite der Verbindungsgeraden $s_2'$ oder $E_1S$, welche den Endpunkt $E_1$ der ersten Kurvenabschnitts $K_1$ mit der Achse S des Zangengelenks Z verbindet. Von dieser Geraden $s_1'$ oder AS hat der Krümmungsmittelpunkt $M_1$ einen Abstand $q_1$, der zumindest annähernd gleich groß ist wie der Abstand q, den der Mittelpunkt M beim vorbeschriebenen Ausführungsbeispiel von der Verbindungsgeraden ES = $s_2$ aufweist.

Dieser Kurvenabschnitt $K_1$ hat einen Krümmungsradius $R_1$ der etwa einem Drittel der Länge der Geraden $S_2'$ entspricht. Der sich absatzlos an den Abschnitt $K_1$ anschließende Kurvenabschnitt $K_2$ hat einen Krümmungsradius $R_2$, der etwa doppelt so groß ist wie der Krümmungsradius $R_1$. Dabei liegt der Krümmungsmittelpunkt $M_2$ dieses Kurvenabschnittes $K_2$ auf der dem Hebelarm 12 des Griffhebels 10 gegenüberliegenden Seite der Verbindungsgeraden $s_2'$, welche den Endpunkt E des Kurvenabschnittes $K_2$ mit der Achse S des Zangengelenks Z verbindet.

Man erkennt auch hierbei, daß der Abstand bzw. die Länge der Geraden $s_0'$ = AS, welche den Anfangspunkt A des Kurvenabschnittes $K_1$ mit der Achse S verbindet, kleiner ist als die Länge der Geraden $s_1'$ = $E_1S$ und diese wiederum kleiner ist als die Länge der Geraden $s_2'$ = ES. Mit einer solchen Anordnung kann man, wie aus Fig. 17 erkennbar ist, unterschiedliche Schneidkraftprogressionen an den Schneiden 4', 27' erzielen. Während im Bereich des Kurvenabschnitts $K_1$ mit dem wesentlich kleineren Krümmungsradius $R_1$ eine wesentlich höhere Progression der Schneidkraft $P_0$ und $P_2$ während der Bewegung im Bereich des Schließwinkels $\alpha_1$ stattfindet, ist diese Progression im Bereich des Kurvenabschnitts $K_2$ mit dem wesentlich größeren Krümmungsradius $R_2$ auch erheblich geringer.

Während sich der Hebelarm $a_1$ am Ende der Winkelbewegung $\alpha_1$ etwa um vier Fünftel auf ein Fünftel verringert, beträgt die Verringerung von $a_1$ auf $a_2$ während der restlichen halben Schließbewegung von $\alpha_2$ bzw. Schneidbewegung nur etwa ein Drittel. Das bedeutet, daß die Kraftzunahme an den Schneiden 4', 27' während der ersten Hälfte der Schneidbewegung um das Fünffache zunimmt und während der letzten Hälfte der Schneidbewegung lediglich um das Dreifache zunimmt.

Man erkennt daraus, daß bei diesem Konstruktions- und Funktionsprinzip sehr variable, den jeweiligen Bedürfnissen angepaßte Progressionsverläufe der Kraftänderungen wählbar sind, ohne daß die Schneidzange dadurch eines größeren Herstellungsaufwandes bedarf. Dabei dürfte auch von wesentlichem Vorteil sein, daß man die jeweils benötigten Kurvenbahnen sowohl rechnerisch als auch zeichnerisch leicht ermitteln kann.

Um die abgeschnittenen Teile eines Drahtes 40, einer Schraube od. dgl. im Zeitpunkt des Abtrennens zumindest eingeschränkt festzuhalten, sind in unmittelbarer Nähe der beiden Schneiden 4' und 27' an den beiden, jeweils ein Schneidplättchen 4 bzw. 27 aufweisenden Schneidarmen 3 und 26, elastische Drahthalter 45, 46 sich gegenüberliegend angeordnet.

Damit die beiden Griffhebel 1 und 10 in der in Fig. 1 dargestellten gespreizten Lage gehalten bzw. nach jedem Schneidvorgang wieder in diese Lage gebracht werden, sind an ihren Innenseiten jeweils Blattfedern 50 und 51 angeordnet, die an ihren Enden durch ein Gelenk 52 miteinander verbunden sind. Die anderen Enden dieser beiden Blattfedern 50 und 51 sind jeweils durch Schrauben an den Innenseiten der Hebelarme 2 bzw. 12 befestigt.

**Patentansprüche**

1. Schneidzange, insbesondere für chirurgische Zwecke, zum Durchtrennen von Drähten (40), Nägeln, Schrauben oder dgl. mit einem ersten zweiarmigen Griffhebel (1), dessen längerer hinterer Hebelarm (2) als Handgriff ausgebildet ist und dessen kürzerer vorderer Hebelarm (3) an seinem vorderen Endabschnitt mit einer Schneide versehen ist, und mit einem ebenfalls zweiarmigen zweiten Griffhebel (10), der durch ein Zangengelenk (Z) schwenkbar am ersten Griffhebel (1) angelenkt ist und der außer einem zum Handgriff des ersten Griffhebels (1) etwa spiegelbildlich ausgebildeten und angeordneten zweiten Handgriff einen vorderen kurzen Hebelarm (11) aufweist, der über ein Hebelgetriebe mit einem eine zweite Schneide od. dgl. aufweisenden zweiarmigen, am kurzen Hebelarm (3) des ersten Griffhebels (1) angelenkten Schneidhebel (20) derart getrieblich verbunden ist, daß sich die über die Handgriffe auf die Schneiden ausgeübte Schneidkraft mit zunehmender Schließbewegung überproportional ändert, insbesondere ansteigt,

**dadurch gekennzeichnet,**
**daß** der kurze Hebelarm (11) des zweiten Griffhebels (10) in einem radialen Abstand vom Zangengelenk (Z) eine Kurvenbahn ($K_1$, $K_2$) aufweist, über welche dieser kurze Hebelarm (11) während eines Schneidvorgangs auf den ihm zugekehrten Hebelarm (25) des Schneidhebels (20) einwirkt, der zu diesem Zweck mit einem auf der Kurvenbahn ($K_1$, $K_2$) reibungsarm rollenden oder gleitenden Übertragungselements (31, 31') versehen ist, und daß die Kurvenbahn (K, $K_1$, $K_2$) so verläuft, daß sich der radiale Abstand ($a_o$, $a_1$, $a_2$), den die Kraftlinien ($Kl_o$, $KL_1$, $KL_2$) der während einer Schließbewegung des zweiten Griffhebels (10) jeweils im Kraftübertragungspunkt ($E_1$, E) an der Kurvenbahn (K, $K_1$, $K_2$) wirksamen Schneidkraftkomponente von der Achse (S) des Zangengelenks (Z) aufweisen, von einem Maximalwert ($a_0$) am Beginn der Schließbewegung in einen Minimalwert ($a_2$) am Ende der Schließbewegung ändert.

2. Schneidzange nach Anspruch 1, **dadurch gekennzeichnet, daß** die Kurvenbahn ($K_1$) innerhalb des Bewegungswinkels ($\alpha_2$) des zweiten Griffhebels (10) einen stetigen Verlauf aufweist und daß der radiale Abstand ($s_1$) des näher am Zangengelenk (Z) gelegenen Anfangs (A) der Kurvenbahn (K) von der Achse (S) des Zangengelenks (Z) kleiner ist als der radiale Abstand ($s_2$) den das Ende (E) der Kurvenbahn (K) von der Achse (S) des Zangengelenks (Z) aufweist.

3. Schneidzange nach Anspruch 1 oder 2, **dadurch gekennzeichnet, daß** die Kurvenbahn (K) eine zumindest annähernd konstante Krümmung aufweist, deren Krümmungsmittelpunkt (M) auf der vom langen Hebelarm (12) des zweiten Griffhebels (10) abgewandten Seite der das Ende (E) der Kurvenbahn (K) mit der Achse (S) des Zangengelenks (Z) verbindenden Geraden (ES = $s_2$) liegt und deren Krümmungsradius (R) um wenigstens ein Drittel kleiner ist, als die Länge dieser Geraden (ES = $s_2$).

4. Schneidzange nach Anspruch 3, **dadurch gekennzeichnet, daß** der Krümmungsradius (R) der Kurvenbahn (K) zumindest annähernd dem halben Abstand ($s_2$) entspricht, den das Ende (E) der Kurvenbahn (K) von der Achse (S) des Zangengelenks (Z) hat und daß ihr Krümmungsmittelpunkt (M) von der Geraden (ES) einen Abstand (q) aufweist, der kleiner ist als ein Zehntel des Krümmungsradius (R) der Kurvenbahn (K).

5. Schneidzange nach einem der Ansprüche 1 bis 3, **dadurch gekennzeichnet, daß** die Kurvenbahn (K) mit einer zweiten dazu parallelen Kurvenbahn (K') eine Kulissenführung (30) für das Übertragungselement (31, 31') des Schneidhebels (20) bildet, welche die Form eines gekrümmten Langloches aufweist.

6. Schneidzange nach Anspruch 5, **dadurch gekennzeichnet, daß** das Übertragungselement aus einer am Schneidhebel (20) drehbar gelagerten Rolle (31) besteht, deren Durchmesser (d) der Breite (B) der Kulissenführung (30) angepaßt ist.

7. Schneidzange nach Anspruch 3, **dadurch gekennzeichnet, daß** das Übertragungselement aus einem gelenkig auf dem Schneidhebel (20) gelagerten Kulissenstein (31') besteht, dessen Gleitflächen (32, 33) Krümmungen aufweisen, die den Krümmungen der Kurvenbahnen (K und K') der Kulissenführung (30') angepaßt sind.

8. Schneidzange nach Anspruch 1, **dadurch gekennzeichnet, daß** die Kurvenbahn innerhalb des Bewegungswinkels ($\alpha_2$) des zweiten Griffhebels (11) wenigstens zwei Kurvenbahnabschnitte ($K_1$ und $K_2$) mit unterschiedlichen Krümmungsradien ($R_1$, $R_2$) aufweist und daß der Anfang (A) des näher am Zangengelenk (Z) gelegenen Kurvenbahnabschnitts (K1) mit dem kleineren Krümmungsradius ($R_1$) von der Achse (S) des Zangengelenks (Z) einen kleineren radialen Abstand ($s_o$) aufweist als dessen Ende ($E_1$) und das Ende ($E_2$) des zweiten Kurvenbahnabschnitts ($K_2$) von der Achse (S) des Zangengelenks (Z) einen größeren radialen Abstand ($s_2$) aufweist als der mit dem Ende ($E_1$) des ersten Kurvenbahnabschnitts ($K_1$) zusammenfallende Anfang dieses zweiten Kurvenbahnabschnitts ($K_2$).

9. Schneidzange nach Anspruch 8, **dadurch gekennzeichnet, daß** der Krümmungsmittelpunkt ($M_1$) des ersten Kurvenbahnabschnitts ($K_1$) auf der vom zweiten Griffhebel abgewandten Seite der Geraden ($SE_1$) liegt, welche die Achse (S) des Zangengelenks (Z) mit dem Ende ($E_1$) des ersten Kurvenabschnitts ($K_1$) verbindet und daß der dazugehörige Krümmungsradius ($R_1$) kleiner ist als der halbe radiale Abstand ($s_1$) des Endes ($E_1$) des ersten Kurvenbahnabschnitts ($K_1$) von der Achse (S) des Zangengelenks (Z).

10. Schneidzange nach Anspruch 8 oder 9, **dadurch gekennzeichnet, daß** er Krümmungsmittelpunkt ($M_2$) des zweiten Kurvenbahnabschnitts ($K_2$) auf der vom zweiten Griffhebel (10) abgewandten Seite der Geraden ($SE_2$) liegt, welche die Achse (S) des Zangengelenks (Z) mit dem Ende ($E_2$) des zweiten Kurvenabschnitts ($K_2$) verbindet und daß der dazugehörige Krümmungsradius ($R_2$) größer ist als der halbe radiale Abstand ($s_2$) des Endes (E) des zweiten Kurvenbahnabschnitts ($K_2$) von der Achse (S) des Zangengelenks (Z).

**11.** Schneidzange nach einem der Ansprüche 1 bis 10, **dadurch gekennzeichnet, daß** in unmittelbarer Nähe der Schneiden (4', 27') an den beiden jeweils ein Schneidplättchen (4, 27) aufweisenden Schneidarmen (3, 26) elastische Drahthalter (45, 46) sich gegenuberliegend angeordnet sind.

# Fig. 1

# Fig. 2

# Fig. 3

27  27'  40  4'  4

III  III

26  C  3

20  G

VI  b

25  11

30  a₀

33

S  Z

52

II

50

s

12  51

P  2

20  G

13

31

33

15

12

27  27'  4'  4

26  3

45  46

Fig. 4

Fig. 5

Fig. 7

Fig. 6

Fig. 5a

Fig. 8

Fig. 8a

Fig. 8b

Fig. 9

Fig. 10

Fig. 13

Fig. 11

Fig. 14

Fig. 12

Fig. 15

Fig. 16

Fig. 18

Fig. 17